## Europäisches Patentamt

## European Patent Office

## Office européen des brevets

(19)

(11) Publication number: **0 084 053**
**B1**

(12) **EUROPEAN PATENT SPECIFICATION**

(45) Date of publication of patent specification: **09.09.87**

(51) Int. Cl.⁴: **A 61 K 31/56**

(21) Application number: **82902571.7**

(22) Date of filing: **15.07.82**

(86) International application number:
**PCT/US82/00955**

(87) International publication number:
**WO 83/00287 03.02.83 Gazette 83/04**

(54) Pharmaceutical composition and the use of Sn- or Cr-protoporphyrin for the manufacture thereof.

<table>
<tr><td>

(30) Priority: **15.07.81 US 285373**

(43) Date of publication of application:
**27.07.83 Bulletin 83/30**

(45) Publication of the grant of the patent:
**09.09.87 Bulletin 87/37**

(84) Designated Contracting States:
**AT BE CH DE FR GB LI LU NL SE**

(56) References cited:

**Biochimica et Biophysica Acta, vol. 673, March 1981, Maines, Zinc-Protoporphyrin is a selective inhibitor of heme oxygenase activity in the neonatal rat. pages 339-350**

The file contains technical information submitted after the application was filed and not included in this specification

</td><td>

(73) Proprietor: **THE ROCKEFELLER UNIVERSITY**
**1230 York Avenue**
**New York, NY 10021 (US)**

(72) Inventor: **Kappas, Attalah**
**Griswold Road**
**Rye, NY 10580 (US)**
Inventor: **Drummond, Goerge**
**304 West 75th Street**
**New York, NY 10023 (US)**

(74) Representative: **Weinhold, Peter, Dr. et al**
**Patentanwälte Dr. V. Schmied-Kowarzik Dipl.-Ing. G. Dannenberg Dr. P. Weinhold Dr. D. Gudel Dipl.-Ing. S. Schubert Dr. P. Barz**
**Siegfriedstrasse 8**
**D-8000 München 40 (DE)**

</td></tr>
</table>

Courier Press, Leamington Spa, England.

## Description

Iron protoporphyrin, also called heme, or more accurately ferroprotoporphyrin IX, is the specific porphyrin isomer found in mammalian whole blood. Heme is an essential component in the respiratory chain and in the energy transfer reactions that take place in the mammalian body. Heme is synthesized and degraded by known metabolic routes involving known enzymatic reactions.

In the normal course of degradation, heme first undergoes a ring opening reaction to form biliverdin which then converts to bilirubin. Bilirubin is toxic, but normally this toxicity is not manifested since biliverdin rapidly combines with albumin and is transported to the liver. In the liver, the protein is released and the bilirubin is converted to the corresponding diglucuronide by reaction with uridine diphosphoglucuronate. The diglucuronide is soluble and excreted.

A number of synthetic analogs of iron protoporphyrin IX are known. They are commercially available or are readily synthesized by known methods. These include, for example, platinum, zinc, nickel, cobalt, copper, silver, manganese, chromium and tin protoporphyrin IX. For convenience herein, these compounds are referred to generically as Me-protoporphyrin, were Me stands for metal, and specifically by utilizing the chemical symbol for the metal such as Cr-protoporphyrin and Sn-protoporphyrin for the chromium and tin compounds respectively.

One of the more difficult aspects of the toxicity of bilirubin is the so-called "jaundice of the newborn" which arises from an undesirably high concentration of bilirubin in the blood of newborn mammals. In the period immediately after birth, there is a high concentration of heme in mammalian blood. There is also a high degree of heme oxygenase activity. This enzyme is essential to the metabolism of heme to bilirubin. The combination of factors leads to rapid metabolism of large amounts of heme and results in high concentrations of bilirubin in the body fluids of neonates. Since bilirubin is yellow, the infant appears yellow. Hence, the name "jaundice of the newborn".

Free bilirubin is fat-soluble and readily crosses the blood-brain carrier causing extensive and serious brain damage. One manifestation of the toxicity of bilirubin is kernicterus or bilirubin encephalopathy. It may be present clinically as lethargy, rigidity, opisthotonus, high-pitched cry, fever, and convulsions. Death may result. Survivors often have cerebral palsy, frequently of the choreoathetoid type, deafness, mental retardation, and other neurologic defects in infancy or early childhood.

The principal treatments for hyperbilirubinemia have been phototherapy and exchange transfusions. In the former treatment, the patient is exposed to increased intensities of light in the visible range particularly between 420 and 470 nm. This results in increased oxidation of bilirubin or conversion by other mechanisms to products that are not yet fully identified. The treatment is widespread, particularly in the United States, but is not regarded as fully satisfactory since there are many as yet unanswered questions concerning its safety and effectiveness.

The more traumatic procedure of exchange transfusion is indicated with full-term infants when there is an unconjugated bilirubin level of 20 mg/dl. With premature infants, the transfusion may be considered necessary at concentrations as low as 9 mg/dl. It is now generally accepted that concentrations that do not exceed 2.0 mg/dl can be tolerated without unnecessary damage to the infant. However, it appears clearly to be desirable to keep the level of this highly toxic chemical lower than 2 mg/dl if possible.

Increased concentrations of bilirubin often appear in the blood of adult humans and other mammals with sickle cell anemia, thalassemia, and other congenital anemias as well as with animals and humans with acquired liver diseases. With such individuals, the concentration of bilirubin rarely reaches the high levels observed with neonates. It does, however, reach levels that may be toxic and should be controlled.

The article of M. D. Maines "Zinc-Protoporphyrin is a Selective Inhibitor of Heme Oxygenase Activity in the Neonatal Rat" in Biochimica et Biophysica Acta, 673 (1981) 339—350, is directed to studies to examine the liver, spleen and kidney heme oxygenase activity in the rat, and also to investigate the response of said enzyme to several metalloporphyrin complexes. These studies are based on experiments in which the animals were killed and pieces of various organs were studied for their biochemical properties, i.e. they were not real *in vivo* studies. They cannot be used to predict the actions of metalloporphyrins in the living mammals. It has been found that the quantitative or sometimes even the qualitative effect of the Me-protoporphyrins in the living mammal is very different from that found in tests of the type mentioned above.

It is desirable, therefore, to provide available compositions for inhibiting the metabolism of heme so as to decrease the rate at which it degrades and the rate at which bilirubin accumulates in the blood.

It has now been discovered that the rate at which heme metabolizes can be decreased in mammals by administration of an effective amount of a synthetic Me-protoporphyrin. The used compounds are Sn-protoporphyrin and Cr-protoporphyrin. Both of these compounds are readily synthesized in pure form at reasonable costs and are therefore readily available. They are water-soluble and may be compounded with a variety of pharmaceutically acceptable carriers. The tin compound appears to be the more active.

To determine the efficacy of Sn-protoporphyrin for controlling the rate of heme metabolism, neonatal rats were injected subcutaneously at birth, and at 12, 24, 48, and 72 hours postnatally with 0.1 ml solutions of Sn-protoporphyrin. To prepare the solutions for parenteral injection, the material was taken up in a small volume of 0.2 N sodium hydroxide, adjusted to pH 7.4 with one normal hydrochloric acid and made up to final volume with 0.9% sodium chloride. The compositions as prepared and used contained sufficient

**0 084 053**

Sn-protoporphyrin to provide about 100 µmol/kg body weight in each 0.1 ml injection. Control neonates received an equivalent volume of saline at each time point. Groups of neonates (6—30 animals per group) were sacrificed at selected time intervals.

The treated animals and the controls were assayed for heme oxygenase activity and bilirubin concentration. Since heme oxygenase is an essential component in the catabolism of heme, an increase in heme oxygenase activity is indicative of an increase in the rate of heme catabolism. Heme oxygenase was assayed as described by Maines and Kappas in *J. Biol. Chem.*, 253, 2321—2326 (1978). Total bilirubin in serum was estimated by the method of Roth, *Clin. Chem. Acta.*, *17*, 487—492 (1967).

In a six-week study, it was found that, with controls, hepatic heme oxygenase levels in neonates rose rapidly, reaching levels four-fold above those characteristic of adult liver before gradually declining to normal adult levels between days 21 and 42. In the Sn-protoporphyrin-treated neonates, the administration of the active agent completely prevented the normal marked increase in hepatic heme oxygenase activity after birth. Indeed, in these neonates, the heme oxygenase activity declined immediately (1 day) to levels less than half the levels of the controls. They remained well below the control levels for about three weeks, and then rose gradually to finally attain the normal control levels at about day 42.

Sn-protoporphyrin administration resulted in an immediate and significant lowering of serum bilirubin levels in treated animals. This low level of bilirubin concentration continued until about the fourth day and then remained substantially the same as the controls throughout the balance of the 42-day study period.

Similar studies were conducted with neonatal rats treated subcutaneously with 2 and 10 micromoles per kilogram of Cr-protoporphyrin soon after birth. Various organs were analyzed for heme oxygenase activity and bilirubin concentration. The results were compared with controls each of which were treated subcutaneously with 0.1 ml of saline.

The results for heme oxygenase activity in liver, kidney and spleen in nanamoles heme oxygenase per milligram microsomal protein per hour are shown in Table 1.

It is clear from a review of Table 1 that the effect of the administration of Cr-protoporphyrin is to cause an immediate decrease in heme oxygenase. Similar results were observed with serum bilirubin as shown in Table 2 at page 7 in which the concentration is expressed in milligrams per deciliter.

TABLE 1

|  | Time (days) | | | | | | | | |
|---|---|---|---|---|---|---|---|---|---|
|  | 1 | 2 | 3 | 5 | 7 | 10 | 14 | 21 | 35 |
| Liver |  |  |  |  |  |  |  |  |  |
| Control | 6.01 | 7.78 | 8.96 | 10.13 | 6.87 | 6.38 | 4.33 | 2.64 | 2.60 |
| 2 µmol/kg | — | 5.81 | 5.69 | 5.36 | 4.42 | 3.62 | 2.24 | 1.94 | 2.38 |
| 10 µmol/kg | — | 1.67 | 1.43 | 1.22 | 0.85 | 0.85 | 0.78 | 0.56 | 1.23 |
| Kidney |  |  |  |  |  |  |  |  |  |
| Control | 2.59 | 2.14 | 2.13 | 1.28 | 2.64 | 2.84 | 1.31 | 1.05 | 0.94 |
| 2 µmol/kg | — | 0.36 | 0.72 | 0.94 | 1.17 | 1.67 | 0.74 | 0.61 | 0.58 |
| 10 µmol/kg | — | 0 | 0.42 | 0.59 | 0.48 | 0.34 | 0.41 | 0.16 | 0.45 |
| Spleen |  |  |  |  |  |  |  |  |  |
| Control | 6.25 | 8.19 | 15.05 | 16.20 | 13.03 | 15.05 | 16.76 | 9.12 | 9.63 |
| 2 µmol/kg | — | 7.33 | 11.13 | 15.13 | 11.18 | 13.87 | 16.62 | 10.28 | 15.55 |
| 10 µmol/kg | — | 3.97 | 4.62 | 2.76 | 6.19 | 9.53 | 10.34 | 8.06 | 13.72 |

TABLE 2

| Serum Bilirubin | 1 | 2 | 3 | 5 | 7 | 10 | 14 |
|---|---|---|---|---|---|---|---|
| Control | 0.39 | 0.48 | 0.42 | 0.31 | 0.33 | 0.46 | 0.38 |
| 2 µmol/kg | — | 0.39 | 0.33 | 0.28 | 0.32 | 0.33 | 0.40 |
| 10 µmol/kg | — | 0.43 | 0.33 | 0.24 | 0.30 | 0.33 | 0.28 |

Studies were carried out with adult rats treated with Sn-protoporphyrin to determine the effect on heme oxygenase activity and bilirubin production. Three rats weighing about 230 grams each were injected subcutaneously with 50 µmol/kg of Sn-protoporphyrin in isotonic saline at pH 8. Three controls were similarly treated with isotonic saline. The rats were sacrificed sixteen hours after treatment. The microsomal protein of the separate livers and of the three combined kidneys was estimated and heme oxygenase activity determined according to the method of Maines and Kappas. This procedure measures the heme oxygenase activity by determining the amount of bilirubin produced. The results are shown in Table 3. The concentration unit for bilirubin is nanamoles of bilirubin formed per milligram protein per hour.

3

TABLE 3

| | Liver | Kidney (combined) |
|---|---|---|
| Control 1 | 2.19 | |
| Control 2 | 3.29 | 1.24 |
| Control 3 | 2.39 | |
| Experiment 1 | 0.95 | |
| Experiment 2 | 0.95 | 0.055 |
| Experiment 3 | 1.53 | |

It is readily apparent from the above that Sn-protoporphyrin is extremely effective for reducing bilirubin production and heme oxygenase activity in adult mammals.

With neonate mammals, the therapeutic compositions of this invention will be administered promptly after birth at a dosage of from 10 to 25 μmol/kg of body weight in animals and 0.1 to 2.5 μmol/kg of body weight in humans. While any of the usual parenteral routes may be employed, intramuscular or intravenous injection is preferred. Normally, one injection will suffice to maintain the bilirubin concentration at a desired low level until the neonate reaches the age where the metabolism of heme is in balance. It is preferred, however, to monitor the serum bilirubin concentration and to utilize a booster dose, if necessary. The physician or veterinarian will select the appropriate dosage. It will normally be within the above range, but may be varied to deal with specific conditions.

With adults afflicted with sickle cell anemia or another condition resulting in increased bilirubin concentration, the dosage unit is normally smaller, since in all but the most acute situations, the bilirubin concentration is not as high as in neonates. The standard dosage with adults will normally be from about 0.05 to 1 μmol/kg of body weight.

Thus, a subject matter of the present invention is the use of Sn-protoporphyrin or Cr-protoporphyrin for the manufacture of a pharmaceutical composition for decreasing the concentration of serum bilirubin in mammals.

Another subject matter of the invention is a pharmaceutical composition for decreasing the concentration of serum bilirubin in mammals containing an effective amount of Sn-protoporphyrin or Cr-protoporphyrin in buffered isotonic aqueous solution at pH 7 to 8.

According to a special embodiment of the present invention the pharmaceutical composition contains 100 to 2500 μmol of Sn-protoporphyrin or Cr-protoporphyrin per liter of the buffered isotonic aqueous solution.

In another special embodiment of this invention the pharmaceutical composition is subdivided into dosage units, containing 0.1 to 2.5 μmol/ml of Sn-protoporphyrin or Cr-protoporphyrin.

In order to have appropriate dosage units available, the compositions of this invention will normally be prepared in bulk at concentrations of from 100 to 2500 μmol/liter of Sn-protoporphyrin or Cr-protoporphyrin of buffered isotonic aqueous solution at pH 7 to 8.

The usual pharmaceutical carriers are isotonic aqueous salt or glucose solutions buffered to a pH of about 7 to 8, normally 7.4.

A further subject matter of the invention is a pharmaceutical composition containing an effective amount of Sn-protoporphyrin or Cr-protoporphyrin in form of a lyophilized powder, which powder upon reconstitution with water gives a composition according to claims 2—4.

Thus, therapeutic compositions can also be provided in lyophilized powder form to be reconstituted with water to the clinical dosage. For example, the selected Me-protoporphyrin can be taken up in 0.1 molar phosphate or other pharmaceutically acceptable buffer containing sufficient saline or other solute to form isotonic solutions, and freeze-dried to form a powder. The powder can be reconstituted with distilled water or saline solution to provide solutions containing 0.1 to 2.5 μmol/ml of Me-protoporphyrin per milliliter of solution.

**Claims**

1. The use of Sn-protoporphyrin or Cr-protoporphyrin for the manufacture of a pharmaceutical composition for decreasing the concentration of serum bilirubin in mammals.

2. A pharmaceutical composition for decreasing the concentration of serum bilirubin in mammals containing an effective amount of Sn-protoporphyrin or Cr-protoporphyrin in buffered isotonic aqueous solution at pH 7 to 8.

3. A pharmceutical composition as in claim 2 containing 100 to 2500 μmol of Sn-protoporphyrin or Cr-protoporphyrin per liter of the buffered isotonic aqueous solution.

4

4. A pharmaceutical composition as in claim 3 wherein it is subdivided into dosage units, containing 0.1 to 2.5 µmol/ml of Sn-protoporphyrin or Cr-protoporphyrin.

5. A pharmaceutical composition containing an effective amount of Sn-protoporphyrin or Cr-protoporphyrin in form of a lyophilized powder which powder upon reconstitution with water gives a composition according to claims 2—4.

**Patentansprüche**

1. Die Verwendung von Sn-Protoporphyrin oder Cr-Protoporphyrin zur Herstellung einer pharmazeutischen Zusammensetzung zum Senken der Konzentration an Serum-Bilirubin bei Säugern.

2. Eine pharmazeutische Zusammensetzung zum Senken der Konzentration an Serum-Bilirubin bei Säugern, enthaltend eine wirksame Menge an Sn-Protoporphyrin oder Cr-Protoporphyrin in gepufferter isotonischer wässriger Lösung bei pH 7 bis 8.

3. Eine pharmazeutische Zusammensetzung gemäß Anspruch 2, enthaltend 100 bis 2500 µMol Sn-Protoporphyrin oder Cr-Protoporphyrin pro Liter der gepufferten isotonischen wässrigen Lösung.

4. Eine pharmazeutische Zusammensetzung gemäß Anspruch 3, die in Dosiseinheiten unterteilt ist, welche 0,1 bis 2,5 µMol/ml Sn-Protoporphyrin oder Cr-Protoporphyrin enthalten.

5. Eine pharmazeutische Zusammensetzung, enthaltend eine wirksame Menge an Sn-Protoporphyrin oder Cr-Protoporphyrin in Form eines lyophilisierten Pulvers, das nach Rekonstituieren mit Wasser eine Zusammensetzung gemäß Anspruch 2 bis 4 ergibt.

**Revendications**

1. Emploi de Sn-protoporphyrine ou de Cr-protoporphyrine pour la préparation d'une composition pharmaceutique destinée à diminuer la concentration de la bilirubine dans le sérum chez les mammifères.

2. Composition pharmaceutique destinée à diminuer la concentration de la bilirubine dans le sérum chez les mammifères, contenant une quantité efficace de Sn-protoporphyrine ou de Cr-protoporphyrine dans une solution aqueuse isotonique tamponnée à un pH de 7 à 8.

3. Composition pharmaceutique selon la revendication 2 contenant 100 à 2500 µmoles de Sn-protoporphyrine ou de Cr-protoporphyrine par litre de solution aqueuse isotonique tamponnée.

4. Composition pharmaceutique selon la revendication 3 subdivisée en unités posologiques, contenant 0,1 à 2,5 µmoles/ml de Sn-protoporphyrine ou de Cr-protoporphyrine.

5. Composition pharmaceutique contenant une quantité efficace de Sn-protoporphyrine ou de Cr-protoporphyrine sous forme d'une poudre lyophilisée, laquelle, par reconstitution avec de l'eau, donne une composition selon les revendications 2 à 4.